# EUROPÄISCHE PATENTSCHRIFT

(11) **EP 0 637 749 B1**
(45) Veröffentlichungstag und Bekanntmachung des Hinweises auf die Patenterteilung: **23.10.2002**
(21) Anmeldenummer: 94111934.9
(22) Anmeldetag: 30.07.1994
(51) Int. Cl.: G01N 33/487, G01N 33/52, G01N 21/77, G01N 21/86, G01N 35/00

(54) **System zur Analyse von Probenflüssigkeiten**
System for analysing liquid samples
Système d'analyse des échantillons liquides

(30) Priorität: 05.08.1993 DE 4326339
(43) Veröffentlichungstag der Anmeldung: 08.02.1995
(73) Patentinhaber: Roche Diagnostics GmbH, 68298 Mannheim (DE)
(72) Erfinder: Harttig, Herbert, Dr., D-67122 Altrip (DE); Gentsch, Susanne, D-26441 Jever (DE); Schmidt, Elmar, Dr., D-68167 Mannheim (DE); Schüssler, Rudolf, D-68623 Lampertheim (DE)

(56) Entgegenhaltungen:
- EP-A- 0 585 744
- GB-A- 2 096 314
- US-A- 4 218 421
- PATENT ABSTRACTS OF JAPAN vol. 013, no. 073 (P-830), 20.Februar 1989 & JP-A-63 259469 (FUJI PHOTO FILM CO LTD), 26.Oktober 1988,

## Beschreibung

Die vorliegende Erfindung betrifft ein System zur Analyse von Probenflüssigkeiten mit den Elementen ein Meßgerät mit Strahlungsquelle, Strahlungsempfänger, Auswerte- und Anzeigevorrichtung, ein Vorratsbehältnis zur Aufnahme von Testelementen, in dem die Testelemente spiralförmig angeordnet und einzeln versiegelt sind

Weiterhin betrifft die Erfindung ein Verfahren zur Analyse von Probenflüssigkeiten und eine Anordnung von Testelementen, bei der die Testelemente durch ein Band verbunden sind.

Die Erfindung liegt im Bereich der Analytik von Probenflüssigkeiten ohne Reagenzflüssigkeiten, wie sie besonders zur Analyse von Körperflüssigkeiten Verwendung finden. Es sind im Stand der Technik verschiedene Systeme zur Bestimmung von Glucose im Blut bekannt, die sich um eine einfache und hygienische Handhabung bemühen. In den Patentanmeldungen EP-A 0 373 629 und EP-A 0 373 413 werden Apparate beschrieben, die ein Vorratsbehältnis für Elemente zur Probenaufgabe besitzen, das mehrere aufeinanderfolgende Bestimmungen von Testsubstanzen ermöglicht. Die Elemente zur Probenaufgabe besitzen eine Membran zur Abtrennung von Zellen und Partikeln vom Blutserum. In dem durch die Membran hindurchtretenden Blutserum wird mit einem elektronischen Sensor die Konzentration eines Inhaltsstoffes bestimmt. Für das Vorratsbehältnis zur Probenaufgabe werden prinzipiell drei verschiedene Ausführungsformen vorgestellt. In einer ersten Variante befinden sich die Elemente auf einer Diskette, die gedreht wird, um die Elemente nacheinander an den vorgesehenen Ort zur Probenaufgabe zu bewegen. Bei einer zweiten Ausführungsform befinden sich übereinandergeschichtete Streifen, die jeweils eine Membran zur Probenaufgabe tragen, in einem Vorratsgefäß, aus dem sie an den Ort zur Probenaufgabe transportiert werden. Bei einer dritten Variante befinden sich Membranen zur Probenaufgabe auf einem Filmstreifen, der vergleichbar einem fotografischen Film in einem zylindrischen Vorratsgefäß aufgerollt ist. Bei jeder der genannten Varianten besitzen die Elemente zur Probenaufgabe keine Reagenzien, die die Konzentration eines Inhaltsstoffes der Probenflüssigkeit in ein Signal umwandeln, daher sind Sensoren notwendig, die direkt einen zu bestimmenden Inhaltsstoff detektieren.

Der geschilderte Stand der Technik besitzt somit den Nachteil, daß Sensoren direkt mit dem Blutserum, das durch die Membran hindurchtritt, in Kontakt gebracht werden. Dies kann zu einer Kontamination des Sensors führen, die eine Verfälschung des Meßergebnisses zur Folge haben kann und darüber hinaus hygienische Probleme mit sich bringt, wenn beispielsweise Viren durch genannte Membranen hindurchtreten können. Aus Gründen der Hygiene ist es im beschriebenen Stand der Technik ebenfalls nachteilig, daß verwendete Elemente zur Probenaufgabe im System verbleiben.

Aus der Patentanmeldung GB 2096314 ist ein Gerät zur optischen Auswertung von Teststreifen bekannt. Die Teststreifen werden dem System einzeln zugeführt und innerhalb des Systems durch Bestrahlung mit einer Lichtquelle und Detektion reflektierter Strahlung ausgewertet.

Ein Bevorratungssystem für eine Vielzahl von Testelementen ist aus dem US Patent 4,218,421 bekannt. Die Testfelder sind auf einem Band angeordnet, das auf einem ersten Zylinder aufgewickelt ist und das zum Zurverfügungstellen von Testfeldern abgewickelt wird. Gebrauchte Testelemente werden auf eine zweite Rolle aufgewickelt, die mit der ersten Rolle in einem Gehäuse untergebracht ist. Ein Schutz noch unverbrauchter Testelemente wird bei diesem Vorratsbehältnis durch das Gehäuse erreicht, das einen Verschluß besitzt. In dem Dokument wird keine Einzelversiegelung von Testelementen mit einer Folie beschrieben.

In der japanischen Patentanmeldung 63259469 ist eine Verpackung für analytische Testelemente beschrieben, bei denen diese einzeln zwischen zwei Folien eingesiegelt sind. Zur Entnahme von Testelementen wird die Anordnung zwischen zwei Walzen hindurchgeführt, wobei die Testelemente herausgedrückt werden.

Ein Verpackungssystem für streifenförmige Testelemente ist aus dem US Patent 3,918,910 bekannt. In den Figuren 24, 25 und 26 ist eine Versiegelung der Testelemente zwischen zwei Folien dargestellt. Zur Entnahme eines Testelementes werden die Folien auseinandergezogen, so daß die Testelemente freigesetzt werden.

Der Erfindung lag die Aufgabe zugrunde, ein System zur Analyse von Probenflüssigkeiten zur Verfügung zu stellen, bei dem sich mehrere Testelemente zur Durchführung der Analyse so in dem System befinden, daß sie nacheinander verwendet werden können, ohne daß für jede Bestimmung ein erneutes Befüllen von außen notwendig wäre. Ebenfalls war es Aufgabe der Erfindung ein System zu schaffen, das hygienischen Anforderungen gerecht wird und das durch eine längere Haltbarkeit der Testelemente auch Bestimmungen im Abstand von Wochen mit gleichbleibender Genauigkeit ermöglicht.

Die genannten Aufgaben wurden durch ein System zur Analyse von Probenflüssigkeiten gemäß Anspruch 1 gelöst. Zur Erfindung gehört ebenfalls ein Vorratsbehältnis, und ein Verfahren zur Analyse von Probenflüssigkeiten.

Die Testelemente des erfindungsgemäßen Systems befinden sich in einem Gerät, das eine einfache Durchführung von Analysen erlaubt. Dieses Gerät besitzt Vorrichtungen, mit denen Testelemente aus dem Vorratsbehältnis an einen Ort zur Probenaufgabe transportiert werden. Diese oder weitere Vorrichtungen können ebenfalls dazu dienen, Testelemente an einen Ort zur Messung zu transportieren. In einer bevorzugten Ausführungsform des Systems fallen Ort der Probenaufgabe und Ort der Messung zusammen, was die Handhabung des Systems erleichtert, da kein zusätzlicher Transportvorgang notwendig ist. Mit genannten Transportvorrichtungen kann ebenfalls der Auswurf eines benutzten Testelementes aus dem System erfolgen, wozu jedoch auch zusätzliche Vorrichtungen installiert sein können. In einer bevorzugten Ausführungsform findet der Transport der Testelemente aus einem Vorratsbehältnis heraus mit Hilfe eines Bandes statt, an dem die Testelemente aufgereiht sind. Der Weitertransport der Testelemente in die Probenaufgabe bzw. Meßposition erfolgt mit Hilfe eines Schiebers, der auch mit der Optik starr verbunden ist.

Die Testelemente befinden sich vor ihrer Benutzung in einem Vorratsbehältnis. Die vorliegende Erfindung betrifft ebenfalls Vorratsgefäße zur Verwendung in einem erfindungsgemäßen System, das spiralförmig angeordnete Testelemente enthält. Dieses Vorratsbehältnis besitzt bevorzugt die Form einer 3 bis 20 mm hohen Schachtel. Die Grundfläche dieser Schachtel kann im wesentlichen viereckig oder auch rund bzw. abgerundet sein. Die Anordnung der Testelemente kann durch einen spiralförmigen Gang innerhalb des Vorratsbehältnisses unterstützt werden. Die Testelemente können durch eine seitliche Öffnung aus dem System heraustransportiert werden. Zum Schutz gegen Luftfeuchte ist es vorteilhaft, das Vorratsbehältnis zusammen mit einem Trockenmittel in einen aluminiumkaschierten Polyethylenbeutel eingeschweißt in Verkehr zu bringen.

Durch die spiralförmige Anordnung der Testelemente in dem Vorratsbehältnis können eine Vielzahl von Testelementen platzsparend angeordnet werden. Es ist auf diese Weise außerdem eine aufeinanderfolgende Entnahme von Testelementen möglich, bei der die Testelemente in vorgegebener Orientierung aus dem System entnommen werden. Eine Verhakung der Testelemente wird durch diese Anordnung ebenfalls verhindert.

Das Vorratsbehältnis kann aus Materialien gefertigt sein, die in die notwendige Form gebracht werden können und eine ausreichende mechanische Stabilität besitzen. In Frage kommende Materialien sind beispielsweise Pappe, Metalle, wie Aluminium, Weißblech, Messing usw., und bevorzugt Kunststoffe wie z. B. Polyethylen, Polypropylen, Polymethylmetacrylat und Polystyrol. Die Herstellung der Vorratsbehältnisse kann mit den für die jeweiligen Materialien im Stand der Technik bekannten Verfahren erfolgen. Ein Vorratsbehältnis trägt bevorzugt einen Codestreifen, auf dem chargenspezifische Informationen in gerätelesbarer Form stehen. Hierfür kommen Magnetcodes, Strichcodes oder auch maschinenlesbare Klarschrift in Frage. Beim Einlegen des Vorratsbehältnisses in das entsprechende Fach oder die Schublade des Meßgerätes, wird der Code auf dem Magazin automatisch durch eine im Gerät befindliche Lesevorrichtung eingelesen.

Erfindungsgemäße Testelemente weisen einen lichtdurchlässigen, im wesentlichen zylindrischen, Kunststoffstift auf, der einen Durchmesser von etwa 2 bis 6 mm und eine Länge von etwa 2 bis 15 mm hat. Der Kunststoffstift ist von einem Mantel so umgeben, daß die Stirnseiten im wesentlichen frei bleiben. Als Material für den transparenten Stift eignet sich grundsätzlich jedes für Licht durchlässige Material. Optisch klare Kunststoffe haben sich besonders bewährt. Bevorzugt sind Polymethylmethacrylat und Polycarbonat. Ein entsprechendes Testelement ist Gegenstand der deutschen Patentanmeldung mit dem Aktenzeichen P 4227678.0 bzw. der europäischen patentanmeldung EP-A-0 585 744.

Der Mantel, der den lichtdurchlässigen Stift umgibt, kann ebenfalls ein Kunststoff sein, beispielsweise Polyethylen, Polypropylen oder Polyvinylidenfluorid. Als vorteilhaft hat es sich herausgestellt, wenn die Brechungsindizes von lichtdurchlässigem Stift und Mantel so beschaffen sind, daß für in den lichtdurchlässigen Stift eintretendes Licht die Bedingung der Totalreflexion erfüllt wird. Mögliche Kombinationen für Stift/Mantel sind Polycarbonat/Polyethylen und Polymethylmethacrylat/Polyvinylidenfluorid. Der lichtdurchlässige Stift kann auch von einem aufgedampften Metallüberzug, beispielsweise aus Aluminium, umgeben sein. Der Metallüberzug kann seinerseits mit einem Mantel aus Kunststoff umhüllt werden. Der Mantel umschließt den Kunststoffstift bevorzugt gas- und flüssigkeitsdicht. Bevorzugt ragt der Mantel an den Stirnseiten des Kunststoffstiftes über diesen hinaus.

Auf einer der Stirnseiten des Kunststoffstiftes ist eine Reagenzschicht aufgebracht, die durch Reaktion mit einem zu bestimmenden Analyten ein optisch detektierbares Signal erzeugt. Auf der Reagenzschicht können weitere Schichten aufgebracht sein, die beispielsweise die Abtrennung von Zellen ermöglichen. Die Zusammensetzung von Reagenz und Hilfsschichten können dem Stand der Technik für Teststreifen entnommen werden. In einer bevorzugten Ausführungsform befindet sich oberhalb der Reagenz- und Hilfsschichten eine Metallisierung, die durch Aufdampfen erzeugt wird. Diese Metallschicht übernimmt die Funktion Umgebungslicht, sowie rote Blutfarbe abzuschirmen. Bei einer Dicke von ca. 70 µm ist eine solche Metallschicht, die bevorzugt aus Aluminium besteht, sowohl ausreichend lichtdicht als auch ausreichend durchlässig für Probenflüssigkeiten. Detailliertere Angaben zur Metallisierung einer Reagenzschicht finden sich in der deutschen Patentanmeldung mit dem Aktenzeichen P 4227665.9 bzw. der europäischen Patentanmeldung EP-A-0 584 721.

Mit der überstehenden Mantelfläche des Testelementes, an der Seite, auf der sich das Reagenz befindet, kann das Testelement auf eine im wesentlichen wasserundurchlässige Folie, z. B. eine Aluminium-Folie oder ein Aluminium-Laminat, aufgesiegelt werden. Dies kann z. B. durch Aufschmelzen oder Aufkleben erfolgen. Diese Ausführungsform bietet den Vorteil, daß das Reagenz wasserdampfdicht gegen die Umgebung abgeschlossen ist, was die Haltbarkeit jedes Testelementes über Wochen gewährleistet. Wird die Versiegelung eines Testelementes entfernt, so bleibt die Versiegelung der übrigen Testelemente intakt. Diese Art der Versiegelung wird als Einzelversiegelung bezeichnet. Erfindungsgemäß ist es möglich, die Testelemente auch mit anderen Seiten oder Stellen an das Band bzw. die Folie zu binden. In Fällen, in denen das Band nicht die Funktion einer Versiegelung übernimmt, kann der Befestigungsort relativ frei gewählt werden.

Die Versiegelung kann so erfolgen, daß die versiegelten Testelemente einzeln vorliegen. Bevorzugt sind jedoch zwei oder mehrere Testelemente an unterschiedlichen Orten der wasserundurchlässigen Folie aufgesiegelt, so daß die Testelemente mechanisch miteinander verbunden sind. Besitzt die Folie die Form eines Bandes, so können die Testelemente mit diesem Band spiralförmig aufgewickelt werden. Der Transport der Testelemente kann ebenfalls durch dieses Band erfolgen.

Das Band mit den aufgesiegelten Einzeltesten ist bevorzugt spiralförmig in ein Magazin eingelegt. Um möglichst kurze Transportwege der Testelemente im Meßgerät zu ermöglichen, wird die Orientierung bevorzugt so gewählt, daß das Band auf der Außenseite der Spirale liegt. Durch Einbau einer Stützspirale im Inneren des Vorratsgefäßes kann ein verhakungsfreies Herausziehen des Bandes mit den aufgesiegelten Testelementen erfolgen.

Ein Vorlauf des Bandes, an dem sich die Testelemente befinden, ist an einem Wickelkern befestigt. Dieser Wickelkern wird durch eine Mechanik, z. B. ein Zahnrad, gedreht. Dadurch wird das Band und mit ihm die Testelemente aus dem Magazin gezogen.

Beim Herausziehen eines Testelementes wird dies durch Abziehen des Bandes entsiegelt. Bevorzugt erfolgt dies, indem die Bewegungsrichtung des Bandes durch eine Umlenkvorrichtung geändert wird. Eine Möglichkeit ist es, eine Rolle oder Kante an der Austrittsöffnung des Vorratsbehältnisses zu positionieren, über die das Band läuft.

Ein entsiegeltes Testelement wird in eine Probenaufgabeposition gebracht. Bevorzugt wird das Testelement beim Transport zur Probenaufgabeposition in eine Dichtung geschoben, die den Mantel des Testelementes umfaßt. Auf diese Weise kann ein Eindringen von Probenflüssigkeit in das Innere des Gerätes verhindert werden. Es ist ebenfalls vorteilhaft, wenn die Fassung, die das Testelement während der Probenaufgabe aufnimmt, in leerem Zustand durch eine Klappe gegen das Eindringen von Staub, Spritzwasser, etc. geschützt ist. Eine solche Klappe kann durch das Einschieben des Testelementes aufgestoßen werden.

Die Messung eines Signals, das für die Konzentration an zu bestimmendem Inhaltsstoff charakteristisch ist, erfolgt von der dem Reagenz abgewandten Stirnseite des Testelementes. Die Einstrahlung und Auslesung erfolgt bevorzugt jeweils nahezu senkrecht zur unteren Stirnfläche des lichtdurchlässigen Stiftes. Das Übersprechen von Sender zu Empfänger kann durch eine zwischengestellte Blende reduziert werden. Diese Anordnung kombiniert die Möglichkeiten geringer Baugröße und relativ großer Abstandstoleranz. Ein Sender zur Verwendung in einem erfindungsgemäßen System emittiert elektromagnetische Strahlung bevorzugt im optischen Teil des Spektrums. Aufgrund ihrer geringen Größe und Leistungsaufnahme haben sich lichtemittierende Halbleiterdioden für diesen Zweck besonders bewährt. Für eine erfindungsgemäß bevorzugte Remissionsmessung werden mit Vorteil Halbleiterdioden im roten und infraroten Bereich eingesetzt.

Der Empfänger empfängt Strahlung, die von der dem Sender und Empfänger zugewandten Reagenzschichtseite abgestrahlt wird. Bevorzugt entsprechen die vom Empfänger aufgenommenen Strahlungsfrequenzen im wesentlichen denen, die vom Sender emittiert werden. Es ist jedoch auch möglich, daß sich vom Sender emittierte und vom Empfänger aufgenommene Strahlung in ihrem Frequenzbereich unterscheiden, wenn zum Beispiel Fluoreszenzprozesse in der Reagenzschicht stattfinden. Als Empfänger werden bevorzugt Halbleiterbaulemente, wie Fotodioden oder Fototransistoren eingesetzt, sie können jedoch auch durch andere im Stand der Technik bekannte Empfänger für elektromagnetische Strahlung realisiert werden.

Zur Erfindung gehört ebenfalls ein Verfahren zur Analyse von Probenflüssigkeiten mit einem System mit einer Anordnung von Testelementen, die durch ein Band miteinander verbunden sind und sich in einem Vorratsbehältnis befinden, beinhaltend die Schritte: Transport eines Testelementes mittels des Bandes aus dem Vorratsbehältnis, Öffnen der Versiegelung des Testelementes durch Abziehen des Bandes von dem Testelement, so daß ein Testfeld des Testelementes zugänglich wird, Aufgabe einer Probenflüssigkeit auf das Testelement, Durchführung des Meßprozesses unter Bestrahlung des Testelementes und Messung von reflektierter oder transmittierter Strahlung, Berechnung eines Analyseergebnisses aus der Messung, Auswurf des verwendeten Testelementes aus dem System.

Der Transport eines Testelementes aus dem Vorratsbehältnis an den Ort zur Probenaufgabe kann erfolgen, indem von einer Mechanik ein unbenutztes Testelement an dem Band, das die Testelemente verbindet, herausgezogen wird und es darauffolgend an den Ort zur Probenaufgabe geschoben wird. Das Herausziehen erfolgt beispielsweise indem der Wickelkern des Vorratsbehältnisses gedreht wird oder indem an dem Band, das die Testelemente verbindet, gezogen wird. Bevorzugt wird ein Band in Form einer Folie, das die Testelemente versiegelt so über eine Kante oder Rolle mit kleinem Durchmesser gezogen, daß dabei die Folie von dem Testelement abgelöst wird und dieses zum Aufbringen der Probenflüssigkeit bereit ist. Es ist jedoch auch möglich, daß die Ablösung eines Testelementes und damit das Öffnen der Versiegelung manuell erfolgt. Der Transport eines Testelementes an den Ort zur Probenaufgabe erfolgt bevorzugt mechanisch. Dieser Transportvorgang kann ebenso wie der Entnahmevorgang aus dem Vorratsbehältnis mit der Öffnung der Versiegelung des Testelementes verbunden werden. Die Aufgabe der Probenflüssigkeit auf das Testelement erfolgt in der Regel manuell, wobei sich das Testelement bevorzugt an einer exponierten Stelle des Systems befindet, damit eine Kontamination des Systems weitgehend verhindert wird.

Der Ort der Probenaufgabe und der Ort zur Messung können bei einem erfindungsgemäßen System verschieden sein, so daß ein Transport des Testelementes notwendig ist.

Der Transport des mit der Probenflüssigkeit versehenen Testelementes an den Ort zur Messung erfolgt bevorzugt mechanisch. Besonders bevorzugt ist eine Anordnung, bei der die Messung direkt in der Position zur Probenaufgabe erfolgt. Die Messung kann beispielsweise durch die Probenaufgabe in Gang gesetzt werden, indem bei eingeschaltetem Gerät in zeitlichen Abständen Messungen durchgeführt werden, die eine Detektion der Probenaufgabe möglich machen. Die Messung der von der Reagenzschicht remittierten Strahlung erfolgt darauf bevorzugt nach dem Verstreichen einer Zeitspanne, die abhängig von den verwendeten Testelementen und der durchzuführenden Analyse ist. Es können auch Messungen in zeitlichen Abständen erfolgen und die zeitliche Veränderung des Meßsignals gespeichert werden. Mit einem solchen Prozeß kann sowohl der optimale Zeitpunkt für die Berechnung des Analyseergebnisses als auch die Kinetik der Detektionsreaktion ermittelt werden.

Die Berechnung des Analyseergebnisses aus der Messung kann mit im Stand der Technik bekannten Verfahren der Datenverarbeitung erfolgen. Zuordnungen, die die Ermittlung einer Konzentration aus dem Meßsignal ermöglichen, können im System gespeichert vorliegen. Vorzugsweise liegen Daten vor, die eine Kalibrierung der Messung für die im Vorratsbehältnis enthaltenen Testelemente ermöglichen. Diese Daten können in bereits beschriebenen Varianten auf dem Vorratsbehältnis gespeichert sein oder auch getrennt vorliegen. Besonders bevorzugt ist eine Verknüpfung von Daten der jeweils verwendeten Testelemente und von im System permanent gespeicherten Daten für eine Auswertung der Messung.

Das ermittelte Analyseergebnis kann dem Benutzer auf einem der im Stand der Technik bekannten Displays dargestellt werden. Es ist auch möglich, vorangehende Analyseergebnisse mit zugehörigen Daten, z. B. dem Datum, darzustellen, so daß der Benutzer eine Veränderung der Analyseergebnisse erkennen kann. Dies ist besonders im Bereich der Glucose-Bestimmung vorteilhaft, da der Patient auf diese Weise seinen Glucose-Spiegel überwachen kann.

Ein wichtiges Charakteristikum des erfindungsgemäßen Systems ist der Auswurf benutzter Testelemente, nachdem die Messung durchgeführt wurde. Der Auswurf kann manuell oder bevorzugt mechanisch erfolgen, z. B. indem das Testelement mit einem Schieber, der es an den Ort zur Messung transportiert hat, über einen mechanischen Widerstand hinwegbewegt wird.

Ein erfindungsgemäßes System bietet dem Anwender einige Vorteile gegenüber herkömmlichen Systemen. Durch die Kombination eines Vorratsbehältnisses mit einem Meßgerät können eine Vielzahl von Analysen durchgeführt werden, ohne daß eine Zuführung einzelner Testelemente von außen notwendig ist. Die Handhabung eines erfindungsgemäßen Systems ist auch dadurch vereinfacht, daß der Transport und die Positionierung der Testelemente durch Vorrichtungen des Systems vorgegeben werden kann, so daß Bedienungsfehler auf ein Minimum reduziert werden. Ein Auswurf benutzter Testelemente wird hygienischen Anforderungen gerecht und verringert die Gefahr der Kontamination des Gerätes, die unter anderem auch zu Fehlmessungen führen könnte. Durch ein erfindungsgemäßes Transportband wird auf einfache und effektive Weise die Einzelversiegelung der Testelemente mit ihrem Transport kombiniert, was die Funktionweise des Gerätes vereinfacht. Durch eine erfindungsgemäße Einzelversiegelung der Testelemente können mit dem System analytische Bestimmungen über einen Zeitraum von Wochen mit derselben Füllung des Vorratsbehältnisses bei gleichbleibender Genauigkeit durchgeführt werden.

Ein erfindungsgemäßes System wird durch das folgende Ausführungsbeispiel, dargestellt in den Figuren 1 bis 4, näher charakterisiert.
- Figur 1:: Gerät zur Bestimmung von Glucose und Vorratsbehältnis für Testelemente
- Figur 2:: Vorratsbehältnisse für Testelemente
- Figur 3:: Testelement
- Figur 4:: Verfahrensschritte für den Transport eines Testelementes aus dem Vorratsbehältnis an den Ort zur Probenaufgabe

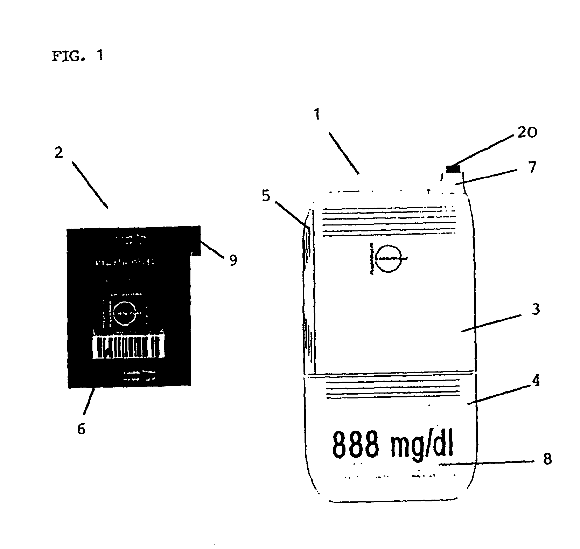

In Figur 1 ist ein Gerät (1) zur Bestimmung von Glucose in Blut und ein zugehöriges Vorratsbehältnis (2) für Testelemente dargestellt. Das Gerät (1) besitzt ein zweiteiliges Gehäuse mit einem oberen Teil (3) und einem unteren Teil (4). An der Seite des Gerätes befindet sich ein Schubfach (5), in welches das Vorratsbehältnis (2) eingeschoben werden kann. Beim Einschieben wird der Strichcode (6) auf der Oberseite des Vorratsbehältnisses (2) durch einen im Gerät (1) befindlichen Strichcodeleser eingelesen. Die in Form des Strichcodes (6) gespeicherten Daten betreffen das Haltbarkeitsdatum der Testelemente und chargenspezifische Informationen zur Kalibrierung des Gerätes (1). An dem Gerät befindet sich ein herausragender Konus (7), der zur Aufnahme eines Testelementes (20) während der Probenaufgabe dient. Durch diese Anordnung kann das Gerät ähnlich wie ein Stift an einen Blutstropfen gezielt herangeführt werden, ohne daß andere Teile des Gerätes mit Blut in Berührung kommen. Zur Darstellung von Analyseergebnissen befindet sich auf der Oberseite des Gerätes (1) eine Flüssigkristallanzeige (8).

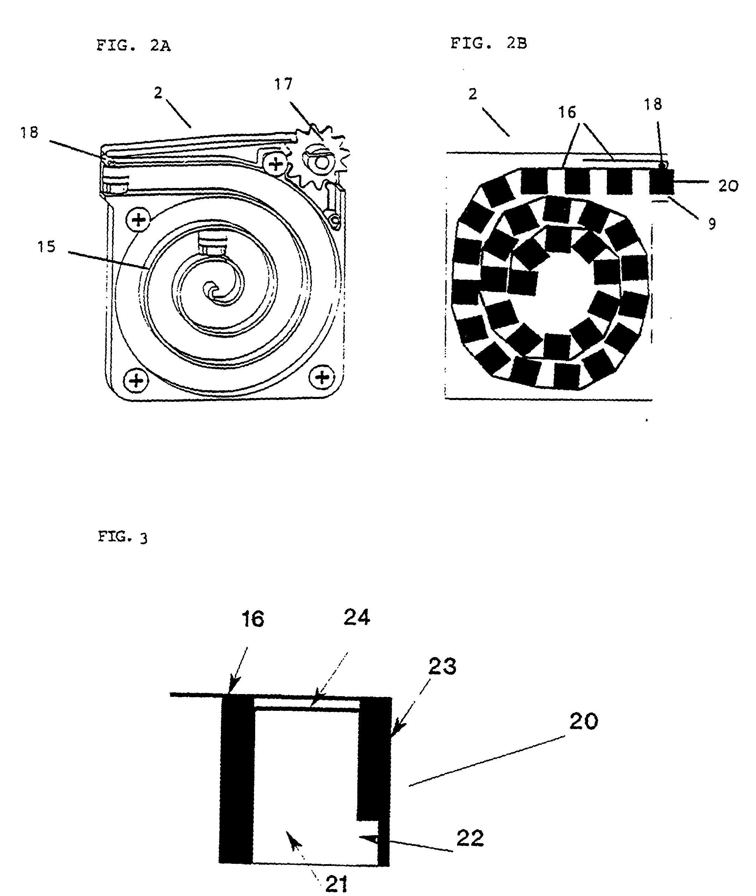

Figur 2 zeigt zwei schematische Darstellungen von Vorratsbehältnissen (2) für Testelemente (20). In Figur 2A ist ein Querschnitt parallel zur Oberseite des Vorratsbehältnisses dargestellt. Eine Spirale (15) im Inneren des Vorratsbehältnisses stabilisiert eine spiralförmige Aufwicklung von Testelementen, wie sie in Figur 2B dargestellt ist. Es ist ebenfalls das Band (16) dargestellt, das sowohl für die mechanische Verbindung der Testelemente als auch zu deren Versiegelung dient. Im konkreten Fall wurde als Band (16) eine Aluminiumfolie eingesetzt. Das Band (16) ist in den Wickelkern (17) eingefädelt. Beim Drehen des Wickelkerns (17) wird das Band (16) über eine Rolle (18) mit kleinem Durchmesser gezogen, was sowohl den Transport der Anordnung von Testelementen in Richtung der Öffnung (9) des Vorratsbehältnisses (2) als auch die Ablösung des Bandes (16) von dem Testelement (20) bewirkt, das durch die Öffnung (9) des Vorratsbehältnisses tritt.

Figur 3 zeigt ein einzelnes Testelement (20) in versiegelter Form. Der lichtdurchlässige Stift (21) besteht aus Polycarbonat und besitzt eine Finne (22), die beim Spritzguß des Stiftes entsteht. Für die erfindungsgemäße Funktion des lichtdurchlässigen Stiftes (21) ist diese Finne (22) von essentieller Bedeutung, da sie gewährleistet, daß fabrikationstechnisch auftretende Strukturstörungen des Kunststoffes in diesen Bereich verlagert werden und somit aus dem Lichtweg ferngehalten werden. Der lichtdurchlässige Stift (21) ist von einem Mantel (23) aus Polyethylen umgeben, das auf der Seite des Bandes (16) über den lichtdurchlässigen Stift (21) herausragt. Da sich die Brechungsindizes von Polycarbonat und Polyethylen stark unterscheiden, wird an der Grenzfläche die Bedingung für Totalreflexion erfüllt. Auf dem lichtdurchlässigen Stift (21) befindet sich ein Testfeld (24), auf welches die Probenflüssigkeit aufgegeben wird. Das Testfeld (24) und die Mantelfläche des lichtdurchlässigen Stiftes (21) sind mit einer dünnen Aluminiumschicht von etwa 70 mm belegt. Der Raum oberhalb des Testfeldes ist durch die Aluminiumfolie (16) wasserdampfdicht gegen die Umgebung abgeschlossen. Die Reagenzschicht des Testfeldes (24) kommt daher nicht direkt mit Luftfeuchte in Berührung.

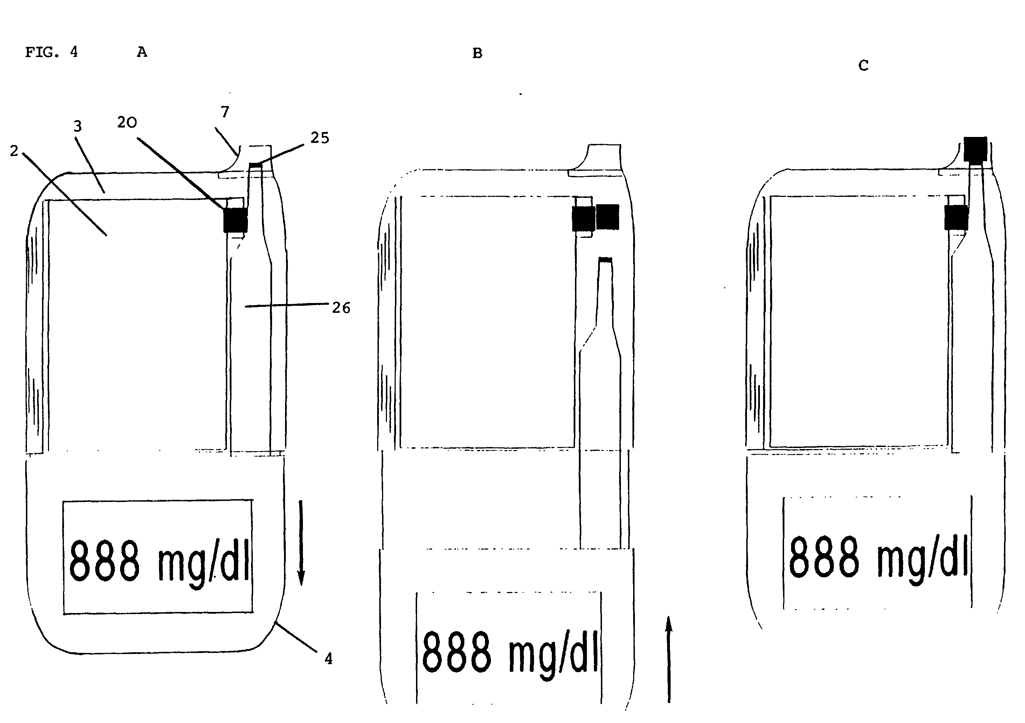

Figur 4 zeigt schematisch den Transport eines Testelementes (20) aus dem Vorratsbehältnis (2) an den Ort zur Probenaufgabe. Zunächst werden unterer Teil (4) und oberer Teil (3) um etwa 30 mm auseinandergezogen (Figur 4A). Nach einem Weg von wenigen Millimetern erfolgt das Einschalten des Gerätes. Auf der Anzeigevorrichtung kann die Anzahl der vorrätigen Testelemente, der letzte Meßwert oder eine Handlungsaufforderung dargestellt werden. Durch Betätigung einer Memorytaste können in dieser Position frühere Ergebnisse aus dem Speicher aufgerufen und angezeigt werden. Nach ca. 10 mm des Schiebeweges wird ein Rastpunkt überschritten, der eine Bewegungsumkehr blockiert. Ab diesem Punkt muß der gesamte Analyseprozeß durchlaufen werden.

Mit dieser Öffnungsbewegung wird auch die Optik (25), die sich an der Oberseite eines Stiftes (26) befindet, am Magazin entlang bewegt, so weit, daß sich die Oberkante der Optik (25) unterhalb der Unterkante der Testelemente befindet (Figur 4B).

Nach dem Rastpunkt wird durch Eingriff einer Zahnstange der Wickelkern (17) des Vorratsbehältnisses (2) gedreht und damit das Band (16) so weit aufgewickelt, daß ein Test aus dem Magazin gezogen wird. Bei diesem Vorgang wird das Testelement durch die Rolle (18) von seiner Versiegelung befreit.

Am Umkehrpunkt der Bewegung wird die Aufwicklung des Bandes ausgekuppelt.

Beim folgenden Zusammenschieben des Gerätes wird das freigelegte Testelement von der Oberkante der Meßoptik in den Konus (7) geschoben (Figur 4C). Der Konus (7) ist so ausgeführt, daß eine Staubschutzblende beim Eindringen des Testelementes wegklappt oder zur Seite geschoben wird. Bei Erreichen der Probeaufgabeposition ragt das obere Ende des Testelements (20) etwa 2,0 bis 2,5 mm aus dem Gerät hervor. Durch den Konus (7) kann die Oberfläche des Testelements etwa 8 bis 12 mm über der Hauptkontur des Gerätes (1) hervorstehen.

Beim Zusammenschieben wird weiterhin das nächste, noch eingesiegelte Testelement unter Abwicklung der bereits aufgewickelten Aluminiumfolie vom Wickelkern (17) bis zu 3 mm zurück in eine definierte Ausgangsposition geschoben. Dadurch wird sichergestellt, daß keine Positionierprobleme in Folge geringfügig unterschiedlicher Wickelstrecken bei anwachsendem Wickeldurchmesser auftreten.

Bei Erreichen der Meßposition, die mit der Probeapplikationsposition identisch ist, wird mit einer Verzögerung von einigen zehntel Sekunden eine Leerwertmessung durchgeführt. Auf der Flüssigkristallanzeige (8) erscheint eine Aufforderung zur Applikation einer Probe.

Als Probe genügt ein Blutstropfen von wenigen µl. Durch Wahl eines hydrophoben Kunststoffes, wie Polypropylen (PP) oder Polyethylen (PE) für den Mantel des Testelementes und PP oder Polyvinylidenfluorid (PVDF) für den Konus (7), wird bewirkt, daß das Blut nicht über die Reagenzschicht (24) hinaus auf das Gerät ausspreitet. Vielmehr bildet ein größeres Probenangebot einen kleinen kugeligen Tropfen auf dem Nachweisbezirk, während die größere Menge auf der Haut des Patienten zurückbleibt. Tropft bei einem stark blutendem Patienten Blut im freien Fall auf die Probeaufgabestelle, so reduzieren die hydrophoben Materialien die Möglichkeit, daß das Blut in das Gerät eindringt und es verschmutzt. Durch enge Passung und gegebenenfalls eine Dichtlippe am Konus (7) kann die Kontaminationssicherheit weiter erhöht werden. Sollte eine Reinigung notwendig werden, kann dies durch eine abnehmbare Ausführung des Konus (7) erleichtert werden.

Die Probenapplikation kann durch Änderung der optischen Eigenschaften des Testelementes (20) vom Gerät selbständig erkannt werden. Die Anzeige des Meßwertes kann durch ein akustisches Signal begleitet werden.

30 bis 60 Sekunden nach Anzeige des Meßergebnisses wird der Benutzer durch Display und Akustiksignal aufgefordert, den gebrauchten Test auszuwerfen. Dazu wird das Gehäuse nach Überwindung einer Sperre weitere 2,5 mm zusammengeschoben. Durch eine Feder erfolgt die Rückführung in den Ausgangszustand.

Mit dem Auswerfen erfolgt gleichzeitig das Ausschalten des Gerätes. Wird am eingeschalteten Gerät keine Funktion betätigt, so erfolgt nach einer Wartezeit von ca. 3 Minuten eine akustische Warnung und danach eine automatische Abschaltung. Ein Neustart kann erst nach erfolgtem Auswurf des gebrauchten Testelementes durchgeführt werden.

### Bezugszeichenliste

- (1): Gerät
- (2): Vorratsbehältnis
- (3): oberer Teil des Gerätes
- (4): unterer Teil des Gerätes
- (5): Schubfach
- (6): Strichcode
- (7): Konus
- (8): Flüssigkristallanzeige
- (9): Öffnung des Vorratsbehältnisses
- 10: -
- (14): ---
- (15): Spirale
- (16): Band
- (17): Wickelkern
- (18): Rolle
- (19): ---
- (20): Testelement
- (21): lichtdurchlässiger Stift
- (22): Finne
- (23): Mantel
- (24): Testfeld
- (25): Optik
- (26): Stift

## Patentansprüche

1. System zur Analyse von Probenflüssigkeiten, beinhaltend
- ein Meßgerät (1) mit Strahlungsquelle, Strahlungsempfänger, Auswerte- und Anzeigevorrichtung, sowie
- ein Vorratsbehältnis (2) zur Aufnahme von Testelementen,
wobei die Testelemente (20) so auf einer Folie (16) aufgesiegelt sind, daß auf den Testelementen befindliche Testfelder (24) durch die Aufsiegelung der Testelemente auf der Folie wasserdampfdicht abgeschlossen sind.

2. System gemäß Anspruch 1, bei dem der Transport eines Testelementes aus dem Vorratsbehältnis mittels der Folie erfolgt.

3. System gemäß Anspruch 1 oder 2, bei dem ein Zugänglichmachen eines Testfeldes (24) des Testelements durch Abziehen der Folie von dem Testelement erfolgt.

4. System gemäß Anspruch 1, bei dem das Testelement einen Stift (21) besitzt, der von einem Mantel (23) umgeben ist, welcher über den Stift hinausragt und auf dem die Folie befestigt ist.

5. System gemäß Anspruch 1 oder 4, bei dem die Testelemente (20) ein Testfeld (24) auf der Stirnseite eines lichtdurchlässigen Stiftes besitzen.

6. System nach Anspruch 1, bei dem die Testelemente durch die Folie (16) miteinander verbunden sind und das System Vorrichtungen (17, 18) besitzt, mit denen die durch die Folie verbundenen Testelemente durch Aufwickeln der Folie auf einen Wickelkern (17) durch das Vorratsbehältnis (2) transportiert werden und ein aus dem Vorratsbehältnis austretendes Testelement durch eine Rolle (18) oder eine Kante von der Folie (16) abgelöst wird.

7. System nach Anspruch 1, bei dem Strahlungsquelle und Strahlungsempfänger auf der der Probenaufgabe gegenüberliegenden Seite des Testfeldes angeordnet sind.

8. System nach Anspruch 1, das einen Stift (26) mit einer Optik (25) besitzt und der Stift (26) eine Relativbewegung ausführen kann, so daß mit dem Stift (26) ein Transport eines Testelementes von der Öffnung des Vorratsbehältnisses in eine Probenaufgabeposition erfolgen kann.

9. System gemäß Anspruch 1, bei dem sich im Inneren des Vorratsbehältnisses eine Spirale (15) befindet, die eine spiralförmige Anordnung von Testelementen stabilisiert.

10. Vorratsbehältnis (2) für Testelemente (20) zur Analyse von Probeflüssigkeiten, beinhaltend eine Anordnung von Testelementen, die so auf einer Folie (16) aufgesiegelt sind, daß die auf den Testelementen befindlichen Testfelder (24) durch die Aufsiegelung der Testelemente auf der Folie wasserdampfdicht abgeschlossen sind.

11. Vorratsbehältnis gemäß Anspruch 10 mit einer Spirale (15) in seinem Inneren, die eine spiralförmige Anordnung von Testelementen stabilisiert.

12. Vorratsbehältnis gemäß Anspruch 10, **dadurch gekennzeichnet, daß** die Folie auf einem Mantel (23) jedes Testelementes (20) befestigt ist, so daß das Testfeld (24) vor dem Zutritt von Luftfeuchte geschützt ist.

13. Vorratsbehältnis gemäß Anspruch 10, beinhaltend eine Kante oder Rolle (18) über die die Folie gezogen werden kann, um ein Testelement von der Folie abzulösen.

14. Verfahren zur Analyse von Probenflüssigkeiten mit Testelementen (20), die auf einer Folie (16) aufgesiegelt sind und die durch die Folie miteinander verbunden sind, beinhaltend die Schritte:
a) Transport eines Testelementes mittels der Folie (16) aus einem Vorratsbehältnis
b) Öffnen der Versiegelung des Testelementes durch Abziehen der Folie von dem Testelement, so daß ein Testfeld (24) des Testelementes zugänglich wird
c) Aufgabe einer Probenflüssigkeit auf das Testelement
d) Durchführung des Meßprozesses unter Bestrahlung des Testelementes und Messung von reflektierter oder transmittierter Strahlung
e) Berechnung eines Analyseergebnisses aus der Messung
f) Auswurf des verwendeten Testelementes aus dem System

15. Verfahren gemäß Anspruch 14, bei dem in Schritt a) die Folie (16) mittels eines Wickelkernes (17) aufgewickelt wird und in Schritt b) das Abziehen der Folie (16) von dem Testelement dadurch erfolgt, daß die Folie (16) über eine Rolle (18) oder Kante umgelenkt wird.

16. Anordnung von Testelementen (20), bei der die Testelemente so auf einer im wesentlichen wasserdampfundurchlässigen Folie (16) so aufgesiegelt sind, daß die Testfelder (24) der Testelemente durch die Aufsiegelung auf der Folie vor einem Zutritt von Wasserdampf geschützt sind.

17. Anordnung von Testelementen gemäß Anspruch 16, bei der die Folie auf einem Mantel (23) jedes Testelementes (20) befestigt ist.

## Claims

1. System for the analysis of sample liquids comprising:
- a measuring instrument (1) with a source of radiation, radiation receiver, evaluation and display device and
- a storage container (2) for holding test elements
wherein the test elements (20) are sealed onto a foil (16) in such a manner that test zones (24) located on the test elements are sealed in a water-vapour-tight manner by means of the sealing of the test elements on the foil.

2. System as claimed in claim 1, in which a test element is transported from the storage container by means of the foil.

3. System as claimed in claim 1 or 2, in which a test zone (24) of the test element is made accessible by peeling the foil from the test element.

4. System as claimed in claim 1, in which the test element has a pin (21) which is surrounded by a sheath (23) which protrudes above the pin and is attached to the foil.

5. System as claimed in claim 1 or 4, in which the test elements (20) have a test zone (24) on the front side of a transparent pin.

6. System as claimed in claim 1, in which the test elements are linked together by the foil (16) and the system has devices (17, 18) with which the test elements that are linked together by the foil are transported through the storage container (2) by winding the foil onto a spool core (17) and a test element emerging from the storage container is detached from the foil (16) by means of a roller (18) or an edge.

7. System as claimed in claim 1, in which the source of radiation and the radiation receiver are arranged on the side of the test zone that is opposite to that of sample application.

8. System as claimed in claim 1, which has a pin (26) with an optical system (25) and the pin (26) can make a relative movement such that a test element can be transported from the opening of the storage container into a sample application position using the pin (26).

9. System as claimed in claim 1 in which a spiral (15) is located inside the storage container which stabilizes a spiral arrangement of test elements.

10. Storage container (2) for test elements (20) for the analysis of sample liquids comprising an arrangement of test elements which are sealed onto a foil (16) in such a manner that test zones (24) located on the test elements are sealed in a water-vapour-tight manner by means of the sealing of the test elements on the foil.

11. Storage container as claimed in claim 10 containing a spiral (15) in its interior which stabilizes a spiral arrangement of test elements.

12. Storage container as claimed in claim 10, wherein the foil is attached to the sheath (23) of each test element (20) in such a manner that the test zone (24) prevents access of air humidity.

13. Storage container as claimed in claim 10 comprising an edge or roller (18) over which the foil can be pulled in order to detach a test element from the foil.

14. Method for the analysis of sample liquids using test elements that are sealed onto a foil (16) and are linked together by the foil comprising the steps:
a) transporting a test element from the storage container by means of the foil (16)
b) opening the seal of the test element by pulling off the foil (16) from the test element such that a test zone (24) of the test element becomes accessible
c) applying a sample liquid onto the test element
d) carrying out the measuring process while irradiating the test element and measuring the reflected or transmitted radiation
e) calculating an analytical result from the measurement
f) ejecting the used test element from the system.

15. Method as claimed in claim 14 in which in step a) the foil (16) is wound on by means of a spool core (17) and in step b) the foil (16) is pulled off from the test element by guiding the foil (16) over a roller (18) or an edge.

16. Arrangement of test elements (20) in which the test elements are sealed onto a foil (16) which is essentially impermeable to water vapour in such a manner that the sealing onto the foil protects the test zones (24) of the test elements from access of water vapour.

17. Arrangement of test elements as claimed in claim 16 in which the foil is attached to a sheath (23) of each test element (20).

## Revendications

1. Système pour l'analyse d'échantillons liquides, comprenant :
- un appareil de mesure (1) doté d'une source de rayonnement, d'un réceptéur de rayonnement, d'un dispositif d'évaluation et d'affichage, ainsi que
- un récipient de réserve (2) pour recevoir des éléments de test, les éléments de test (20) étant scellés sur une feuille (16) de telle sorte que des champs de test (24) soient enfermés de manière étanche à la vapeur d'eau par le scellement des éléments de test sur la feuille.

2. Système selon la revendication 1, dans lequel le transport d'un élément de test hors du récipient de réserve s'effectue au moyen de la feuille.

3. Système selon la revendication 1 ou 2, dans lequel l'accès au champ de test (24) de l'élément de test est réalisé en arrachant la feuille de l'élément de test.

4. Système selon la revendication 1, dans lequel l'élément de test possède une tige (21) qui est entourée par une enveloppe (23) qui déborde au-dessus de la tige et qui est fixée sur la feuille.

5. Système selon la revendication 1 ou 4, dans lequel les éléments de test (20) possèdent un champ de test (24) sur le côté frontal dune tige transparente à la lumière.

6. Système selon la revendication 1, dans lequel les éléments de test (20) sont reliés les uns aux autres par la feuille (16), le système possédant des dispositifs (17, 18) avec lesquels les éléments de test reliés par la feuille sont transportés dans le récipient de réserve (2) par enroulement de la feuille sur un mandrin d'enroulement (17), un élément de test qui sort du récipient de réserve étant libéré de la feuille (16) par un rouleau (18) ou une arête.

7. Système selon la revendication 1, dans lequel la source de rayonnement et le récepteur de rayonnement sont disposés sur le côté du champ de test situé en face de l'application des échantillons.

8. Système selon la revendication 1, qui possède une tige (26) avec une optique (25), la tige (26) pouvant exécuter un déplacement relatif, de telle sorte qu'un transport d'un élément de test depuis l'ouverture du récipient de réserve jusque dans une position d'application de l'échantillon peut s'effectuer à l'aide de la tige (26).

9. Système selon la revendication 1, dans lequel une spirale (15) qui stabilise un agencement en spirale d'éléments de test se trouve à l'intérieur du récipient de réserve.

10. Récipient de réserve (2) pour éléments de test (20) en vue de l'analyse d'échantillons liquides, contenant un agencement d'éléments de test qui sont scellés sur une feuille (16) de telle sorte que des champs de test situés sur les éléments de test (24) soient enfermés de manière étanche à la vapeur d'eau par le scellement des éléments de test sur la feuille.

11. Récipient de réserve selon la revendication 10, présentant à l'intérieur une spirale (15) qui stabilise un agencement en spirale d'éléments de test.

12. Récipient de réserve selon la revendication 10, **caractérisé en ce que** la feuille est fixée sur une enveloppe (23) de chaque élément de test (20) de telle sorte que le champ de test (24) soit protégé contre la pénétration d'humidité de l'air.

13. Récipient de réserve selon la revendication 10, contenant une arête ou rouleau (18) sur lequel la feuille peut être tirée pour libérer un élément de test de la feuille.

14. Procédé pour l'analyse d'échantillons liquides à l'aide d'éléments de test (20) qui sont scellés sur une feuille (16) et reliés les uns aux autres par la feuille, lequel procédé comprend les étapes consistant à :
a) transporter un élément de test hors d'un récipient de réserve au moyen de la feuille (16),
b) ouvrir le scellement de l'élément de test par arrachement de la feuille de l'élément de test de manière à rendre accessible un champ de test (24) de l'élément de test,
c) application d'un échantillon liquide sur l'élément de test,
d) exécution de l'opération de mesure sous irradiation de l'élément de test et mesure du rayonnement réfléchi ou du rayonnement transmis,
e) calcul d'un résultat d'analyse à partir de la mesure, et
f) expulsion de l'élément de test usagé hors du système.

15. Procédé selon la revendication 14, dans lequel, dans l'étape a), la feuille (16) est enroulée à l'aide d'un mandrin d'enroulement (17) et, dans l'étape b), l'arrachage de la feuille (16) de l'élément de test s'effectue en faisant passer la feuille (16) autour d'un rouleau (18) ou d'une arête.

16. Agencement d'éléments de test (20), dans lequel les éléments de test sont scellés sur une feuille (16) essentiellement imperméable à la vapeur d'eau, de telle sorte que les champs de test (24) des éléments de test soient protégés contre une pénétration de vapeur d'eau par le scellement sur la feuille.

17. Agencement d'éléments de test (20) selon la revendication 16, dans lequel la feuille est fixée sur une enveloppe (23) de chaque élément de test (20).
